(19) Europäisches Patentamt
European Patent Office
Office européen des brevets

(11) EP 4 728 980 A1

(12) **EUROPEAN PATENT APPLICATION**
published in accordance with Art. 153(4) EPC

(43) Date of publication:
22.04.2026 Bulletin 2026/17

(21) Application number: 23941751.2

(22) Date of filing: 04.12.2023

(51) International Patent Classification (IPC):
A61B 5/029 (2006.01)    A61B 5/00 (2006.01)
G16H 50/70 (2018.01)    G16H 50/20 (2018.01)
G06N 3/08 (2023.01)    A61B 5/021 (2006.01)
A61B 5/349 (2021.01)    A61B 5/358 (2021.01)
A61B 5/36 (2021.01)

(52) Cooperative Patent Classification (CPC):
A61B 5/00; A61B 5/021; A61B 5/029; A61B 5/349;
A61B 5/358; A61B 5/36; G06N 3/08; G16H 50/20;
G16H 50/70

(86) International application number:
PCT/KR2023/019829

(87) International publication number:
WO 2024/257970 (19.12.2024 Gazette 2024/51)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 14.06.2023 KR 20230076053

(71) Applicant: Seoul National University Hospital
Seoul 03080 (KR)

(72) Inventors:
• LEE, Hyung Chul
Seoul 05504 (KR)
• JUNG, Chul Woo
Seoul 03080 (KR)
• YANG, Hyun Lim
Seoul 01404 (KR)
• PARK, Seong A
Seoul 03100 (KR)

(74) Representative: Noréns Patentbyrå AB
Box 10198
100 55 Stockholm (SE)

(54) **DEVICE FOR NON-INVASIVELY CALCULATING CARDIAC OUTPUT AND OPERATING METHOD THEREFOR**

(57)    The present invention relates to a device for non-invasively calculating cardiac output and an operation method therefor. The method comprises the steps of: receiving first bio-signal data including an electrocardiogram and a photoplethysmogram of a user; and determining a stroke volume of the user on the basis of the first bio-signal data of the user by using a deep learning model configured to output the stroke volume, with the electrocardiogram and the photoplethysmogram used as inputs.

FIG. 1

## Description

### Technical Field

The present disclosure relates to a device for non-invasively calculating cardiac output and a method of operating the same.

### Background Art

Cardiac output (CO) monitoring is used for hemodynamic diagnosis in high-risk patients. Cardiac output is the product of heart rate (HR) and stroke volume (SV), which refers to the amount of blood pumped by the two ventricles of the heart per unit of time (usually in 'minutes'), and is an index that reflects not only the function of the heart but also the state of the entire circulatory system.

Since cardiac output is used to monitor and optimize systemic oxygenation and drug delivery in high-risk surgical patients, it is important to predict cardiac output.

In the thermodilution-based method, which is the current gold-standard (a test to diagnose the progress of disease) methodology, it is method of calculating cardiac output by inserting a pulmonary artery catheter, which is invasive and has complications such as infection.

The minimally invasive cardiac output calculation method (arterial pressure-based method) proposed after the thermodilution-based method is a method of calculating cardiac output using a minimally invasive arterial blood pressure waveform, which is not a technology used in all patients in intensive care units and operating rooms in hospitals, so its usability is limited.

The background technology of the disclosure was written to facilitate understanding of the present disclosure. It should not be understood that the matters described in background technology of the disclosure exist as prior art.

### Disclosure of Invention

### Technical Problem

Due to the problem of the above-described invasive or minimally invasive cardiac output calculation method, a novel method capable of analyzing and calculating cardiac output using only electrocardiogram and photoplethysmogram, which are completely non-invasive biological signals, is required.

As a result, the inventors of the present disclosure sought to develop a method and a device for performing the same for analyzing the collected non-invasive bio-signal data by being mounted on a bio-signal measuring device such as a patient monitor by analyzing a patient's bio-signal and utilizing a secondary index.

Objects of the present disclosure are not limited to the above-mentioned objects, and other objects, which are not mentioned above, can be clearly understood by those skilled in the art from the following descriptions.

### Solution to Problem

In order to solve the above-described objects, a method of operating a device for non-invasively calculating cardiac output according to an embodiment of the present disclosure is provided. The method is configured to include receiving first bio-signal data including an electrocardiogram and a photoplethysmogram of a user; and determining a stroke volume of the user on the basis of the first bio-signal data of the user by using a deep learning model configured to output the stroke volume, with the electrocardiogram and the photoplethysmogram used as inputs.

According to a feature of the present disclosure, the deep learning model is trained from training data for a plurality of patients, and the training data for the plurality of patients may include a VPG (first derivative PPG) waveform which is a first-order derivative waveform of the photoplethysmogram, and a APG (second derivative PPG) waveform which is a second-order derivative waveform of the photoplethysmogram.

According to another feature of the present disclosure, the training data for the plurality of patients may include PR interval, QRS duration, QRS amplitude, and ST segment extracted from the electrocardiogram of each of the plurality of patients.

According to another feature of the present disclosure, the method may further comprise receiving the user's demographic information data. The deep learning model may be configured to determine a stroke volume of the user by using the user's first bio-signal data, second bio-signal data, and the demographic information data as inputs, the demographic information data may include at least one of the user's age, sex, height, and weight, and the second bio-signal data may further include the VPG waveform, the APG waveform, the PR interval, the QRS duration, the QRS amplitude, the ST segment, and PTT.

In order to solve the above-described objects, a device for non-invasively calculating cardiac output according to an embodiment of the present disclosure is provided.

The device may include a memory, a transceiver receiving at least one of a first bio-signal data including an electrocardiogram and a photoplethysmogram, and the demographic information data, and a processor including a deep learning model, the processor may be configured to preprocess the first bio-signal data, extract second bio-signal data from the preprocessed bio-signal data, and infer a stroke volume using a deep learning model configured to output stroke volume by using the first bio-signal data and the second bio-signal data as inputs.

According to a feature of the present disclosure, the processor may be further configured to slice the first bio-signal data into a predetermined length, extract sampling data from the sliced first bio-signal data, and remove noise data from the first bio-signal data.

According to another feature of the present disclosure, the second bio-signal data may include at least one of a VPG (first derivative PPG) waveform which is a first-order derivative waveform of the photoplethysmogram, a APG (second derivative PPG) waveform which is a second-order derivative waveform of the photoplethysmogram, and a PR interval, QRS duration, QRS amplitude, ST segment extracted from the electrocardiogram, and a Pulse Transit Time (PTT) extracted from the electrocardiogram and the photoplethysmogram.

According to another feature of the present disclosure, the processor is further configured to perform a min-max normalization of the photoplethysmogram and extract the VPG waveform and the APG waveform of the photoplethysmogram, and the VPG waveform and the APG waveform may be input to the deep learning model in the form of time series data having the predetermined length.

According to another feature of the present disclosure, the PR interval, the QRS duration, the QRS amplitude, the ST segment, and the PTT may be input to the deep learning model as an average of each value measured multiple times during a time corresponding to the predetermined length.

According to another feature of the present disclosure, the training data may include the first bio-signal data, the second bio-signal data, stroke volume data, and the demographic information data of a plurality of patients, and the demographic information data may include at least one of age, sex, height, and weight.

According to another feature of the present disclosure, the memory may be configured to store a set of training data matched for each of the plurality of patients.

According to another feature of the present disclosure, the deep learning model may have a CNN (configuration Neural Network) structure including at least one SE (Squeeze and Excitation) block and at least one Residual block.

According to another feature of the present disclosure, the device may comprise the transceiver which are further configured to receive the first bio-signal data and the second bio-signal data of the user corresponding to a period from t(t is a positive integer) seconds to (t+n) seconds, and the processor which are further configured to calculate the stroke volume of the user at the (t+n) seconds by using the deep learning model.

Specific details of other embodiments are included in the description and drawings of the present invention.

## Advantageous Effects of Invention

The present disclosure may calculate a patient's cardiac output using bio-signal data collected in a non-invasive manner.

Specifically, the present disclosure may calculate a patient's cardiac output using only electrocardiogram and photoplethysmogram, which are completely non-invasive bio-signals from which limiting factors in invasive or minimally invasive measurement methods have been removed.

In addition, the present disclosure may analyze high-resolution input data using deep learning technology of a deep neural network structure and trains the same, thereby quickly predicting and outputting a result value, that is, a cardiac output, for the new input data.

The effects according to the present disclosure are not limited to the contents exemplified above, and more diverse effects are included in the present disclosure.

## Brief Description of Drawings

FIG. 1 is a schematic diagram of a device for non-invasively calculating cardiac output according to an embodiment of the present disclosure.

FIG. 2 is a block diagram illustrating the configuration of a cardiac output calculating device according to an embodiment of the present disclosure.

FIG. 3 is a flowchart schematically illustrating a method of calculating cardiac output according to an embodiment of the present disclosure.

FIG. 4 is a graph illustrating input data and output data of a cardiac output calculating device according to an embodiment of the present disclosure.

FIG. 5 is a flowchart illustrating a method of calculating cardiac output according to an embodiment of the present disclosure.

FIG. 6 is a graph illustrating an example of noise of an electrocardiogram waveform and a photoplethysmogram waveform.

FIG. 7 is a graph illustrating a photoplethysmogram waveform, a VPG waveform, and a PPG waveform.

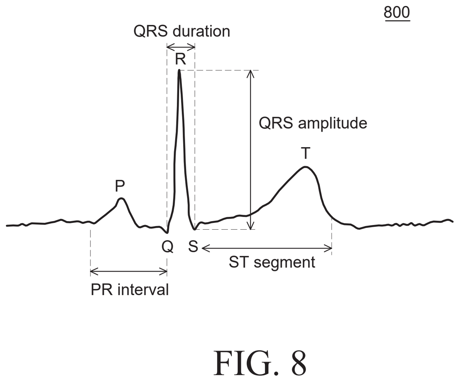

FIG. 8 is a graph illustrating an electrocardiogram waveform and a secondary index extracted from the electrocardiogram waveform.

FIG. 9 is a graph illustrating a secondary index extracted from an electrocardiogram waveform and a photoplethysmogram waveform.

FIG. 10 is a block diagram illustrating an architecture of a Cardiac Output Calculation Model according to an embodiment of the present disclosure.

FIG. 11 is graphs obtained by comparing the predicted value and the actual value of the Cardiac Output Calculation Model according to an embodiment of the present disclosure.

**Best Mode for Carrying out the Invention**

The advantages and features of the present invention, and the methods for achieving them, will become clear with reference to the embodiments described in detail below together with the attached drawings. However, the present invention is not limited to the embodiments disclosed below, but can be implemented in various different forms, and these embodiments are provided only to make the disclosure of the present invention complete and to fully inform a person having ordinary knowledge in the technical field to which the present invention belongs of the scope of the disclosure, and the present invention is defined only by the scope of the claims. In relation to the description of the drawings, similar reference numerals can be used for similar components.

In the present invention, the expressions "have", "can have", "include", or "can include" refer to the presence of a feature (for example, a numerical value, function, operation, or component such as a part) and do not exclude the presence of additional features.

In the present invention, the expressions "A or B", "at least one of A or/and B", or "one or more of A or/and B" can include all possible combinations of the items listed together. For example, "A or B", "at least one of A and B", or "at least one of A or B" can all refer to (1) including at least one A, (2) including at least one B, or (3) including both at least one A and at least one B.

The expressions "first", "second", "firstly", or "secondly", used in the present invention can describe various components, regardless of order and/or importance, and are used only to distinguish one component from another, but do not limit the components. For example, a first user device and a second user device can represent different user devices, regardless of order or importance. For example, without departing from the scope of the rights set forth in the present invention, a first component can be referred to as a second component, and similarly, a second component can be referred to as a first component.

When a component (for example, a first component) is referred to as being "(operatively or communicatively) coupled with/to" or "connected to" another component (for example, a second component), it should be understood that said component can be directly connected to said other component, or can be connected via another component (for example, a third component). Meanwhile, when it is said that a component (for example, a first component) is "directly connected" or "directly coupled" to another component (for example, a second component), it can be understood that no other component (for example, a third component) exists between said component and said other component.

The expression "configured to" as used in the present invention can be used interchangeably with, for example, "suitable for", "having the capacity to", "designed to", "adapted to", "made to", or "capable of, depending on the situation. "The term "configured (or set) to" does not necessarily mean that something is "specifically designed to" in terms of hardware. Instead, in some contexts, the expression "an apparatus configured to" can mean that the apparatus is "capable of" doing something in conjunction with other apparatus or components. For example, the phrase "a processor configured (or set) to perform A, B, and C" can mean a dedicated processor (for example, an embedded processor) for performing the operations, or a general-purpose processor (for example, a CPU or an application processor) that can perform the operations by executing one or more software programs stored in a memory device.

The terms used in the present invention are used only to describe specific embodiments and cannot be intended to limit the scope of other embodiments. The singular expression can include the plural expression unless the context clearly indicates otherwise. Terms used herein, including technical or scientific terms, can have the same meaning as commonly understood by a person of ordinary skill in the art described in the present invention. Terms defined in general dictionaries among the terms used in the present invention can be interpreted as having the same or similar meaning as they have in the context of the relevant technology, and shall not be interpreted in an ideal or excessively formal sense unless explicitly defined in the present invention. In some cases, even if a term is defined in the present invention, it cannot be interpreted to exclude embodiments of the present invention.

Each feature of the various embodiments of the present invention can be partially or entirely combined or combined with each other, and as can be fully understood by those skilled in the art, various technical connections and operations are possible, and each embodiment can be implemented independently of each other or can be implemented together in a related relationship.

For clarity in the interpretation of this specification, the terms used in this specification are defined below.

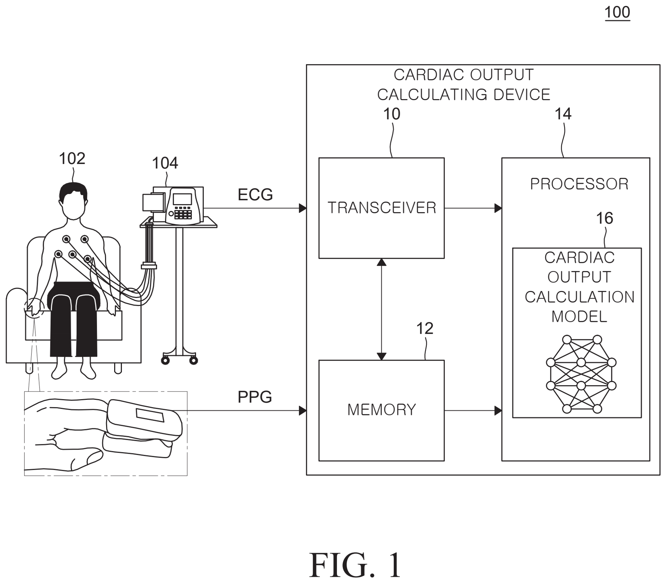

FIG. 1 is a schematic diagram of a cardiac output calculating device according to an embodiment of the present disclosure.

The cardiac output calculating device 100 according to an embodiment of the present disclosure may include a transceiver 10, a memory 12, and a processor 14. The cardiac output calculating device 100 according to an embodiment of the present disclosure may calculate the cardiac output by using an electrocardiogram and/or a photoplethysmogram received through the transceiver 10. The memory 12 may store the received data on the electrocardiogram and/or the photoplethysmogram.

Referring to FIG. 1, the cardiac output calculating device 100 may receive an electrocardiogram of a user 102 obtained through electrocardiogram (ECG) monitoring. The electrocardiogram is recorded in the form of a wavelength by analyzing the electrical activity or electrical conductivity of the heart, and the electrocardiogram monitoring may be performed by attaching electrodes to the skin of a user 102 and monitoring changes in the electrocardiogram displayed in time series through an electrocardiogram measuring device 104.

Further, referring to FIG. 1, the cardiac output calculating device 100 may receive a user's photoplethysmogram obtained through the photoplethysmogram (PPG) monitoring. The method using photoplethysmogram is a method of measuring pulse waves using light, and it is possible to measure the velocity of blood flow and oxygen saturation in the blood by detecting changes in optical properties such as reflection of biological tissues that appear when the volume of blood vessels changes, and absorption transmission ratio or the like. Photoplethysmogram monitoring may be performed by attaching a photoplethysmogram measuring device 106 on a finger of a user, and monitoring a change in photoplethysmogram displayed in time series through the photoplethysmogram measuring device 106.

The cardiac output calculating device 100 according to an embodiment of the present disclosure may be implemented in a personal computer (PC), a data server, or a portable device.

Portable devices may be implemented as a laptop computer, a mobile phone, a smart phone, a tablet PC, a mobile internet device (MID), a personal digital assistant (PDA), an enterprise digital assistant (EDA), a digital still camera, a digital video camera, a portable multimedia player (PMP), a personal navigation device or portable navigation device (PND), a portable game console, an e-book reader, or a smart device. The smart device may be implemented as a smart watch, a smart band, or a smart ring, which are merely examples and does not limit the implementation example of the cardiac output calculating device 100.

The electrocardiogram measuring device 104 and/or the photoplethysmogram measuring device 106 are electronic devices capable of measuring and outputting bio-signals, and may be implemented in a personal computer (PC), a data server, or a portable device, similar to the cardiac output calculating device 100.

The processor 14 of the cardiac output calculating device 100 may include a deep learning model. For example, the processor 14 of the cardiac output calculating device 100 may include a cardiac output calculation model 16.

The cardiac output calculation model 16 may include a neural network (or an artificial neural network). The neural network includes statistical learning algorithms that mimic the nerves of biology in machine learning and cognitive science. The neural network may mean the general model in which artificial neurons (nodes) that form a network by synaptic connections change the strength of synaptic connections through learning, and have problem-solving capabilities. In embodiments of the present disclosure, the cardiac output calculation model 16 may be expressed as a type of neural network and may include a cardiac output calculation algorithm.

A neuron of the neural network may include a combination of weights or biases. The neural network may include one or more layers composed of one or more neurons or nodes. The neural network can infer the desired result from any input by changing the weight of the neuron through learning.

The neural network may include a convolutional neural network (CNN). It will be understood by those skilled in the art that neural networks may include any neural network, but not limited to Deep Neural Network(DNN), Recurrent Neural Network (RNN), perceptron, multilayer perceptron, Feed Forward (FF), Radial Basis Network (RBF), Deep Feed Forward (DFF), Long Short Term Memory (LSTM), Gated Recurrent Unit (GRU), Auto Encoder (AE), Variational Auto Encoder (VAE), Denoising Auto Encoder (DAE), Sparse Auto Encoder (SAE), Markov Chain (MC), Hopfield Network (HN), Boltzmann Machine (BM), Restricted Boltzmann Machine (RBM), Depp Belief Network (DBN), Deep Convolutional Network (DCN), Deconvolutional Network (DN), Deep Convolutional Inverse Graphics Network (DCIGN), Generative Adversarial Network (GAN), Liquid State Machine (LSM), Extreme Learning Machine (ELM), Echo State Network (ESN), Deep Residual Network (DRN), Differentiable Neural Computer (DNC), Neural Turning Machine (NTM), Capsule Network (CN), Kohonen Network (KN) and/or Attention Network (AN).

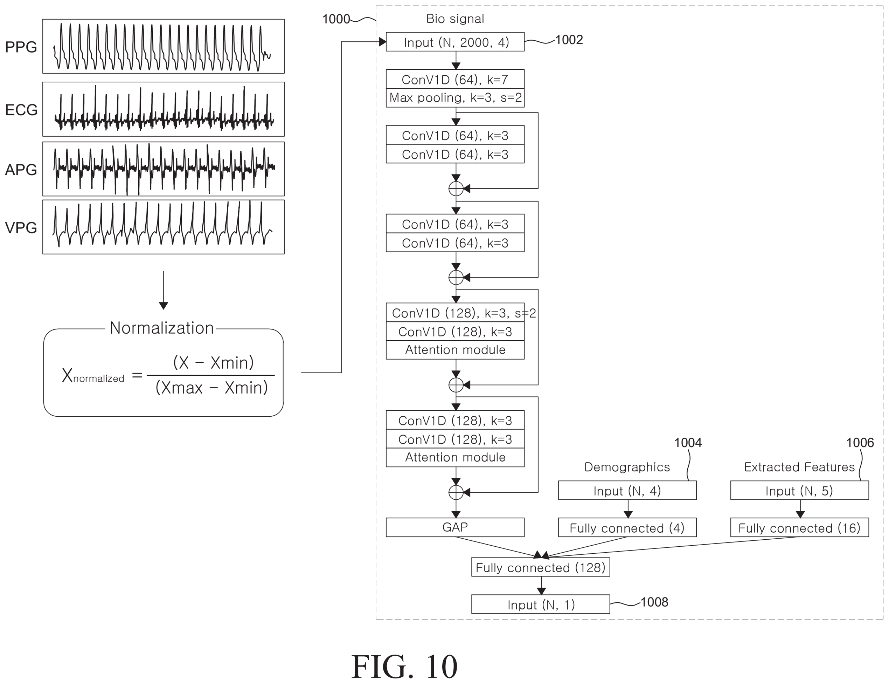

The cardiac output calculation model 16 according to an embodiment of the present disclosure may include a deep learning model including a cardiac output calculation algorithm, and details of the cardiac output calculation model 16 according to an embodiment of the present disclosure will be described below with reference to FIG. 10.

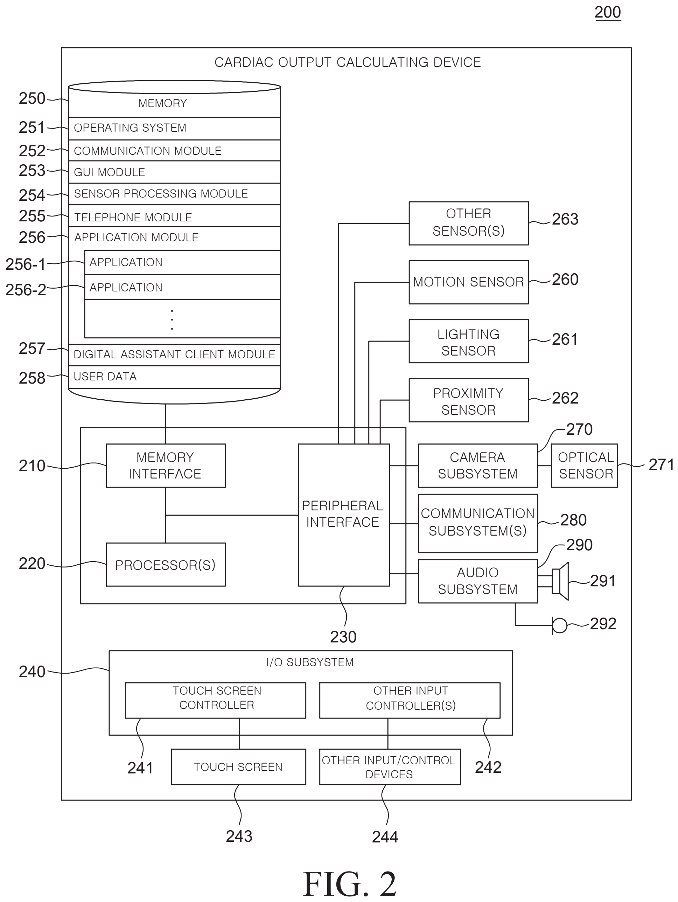

FIG. 2 is a block diagram illustrating the configuration of a cardiac output calculating device according to an embodiment of the present disclosure.

Referring to FIG. 2, a cardiac output calculating device 200 may include a memory interface 210, one or more processors 220, and a peripheral interface 230. The cardiac output calculating device 200 of FIG. 2 may be applied to the cardiac output calculating device 100 of FIG. 1.

Various components in the cardiac output calculating device 200 may be connected by one or more communication buses or signal lines.

The memory interface 210 may be connected to the memory 250 to transmit various data to one or more processors 220. Here, the memory 250 may include at least one type of storage medium among flash memory type, hard disk type, multimedia card micro type, card type memory (for example, SD or XD memory, etc.), RAM, SRAM, ROM, EEPROM, PROM, network storage, cloud, and blockchain database.

In various embodiments, the memory 250 may store at least one of an operating system 251, a communication module 252, a graphical user interface module (GUI) 253, a sensor processing module 254, a telephone module 255, and an application module 256. Specifically, the operating system 251 may include instructions for processing basic system services and instructions for performing hardware operations. The communication module 252 may communicate with at least one of other devices, computer, and server. The graphic user interface (GUI) module 253 may process a graphic user interface. The sensor processing module 254 may process a sensor-related function (e.g., processing voice input received using one or more microphones 292). The telephone module 255 may process telephone -related functions. The application module 256 may perform various functions of a user application, such as electronic messaging, web browsing, media processing, searching, imaging, and other processing functions. In addition, the cardiac output calculating device 200 may store one or more software applications 256-1, 256-2 associated with any one type of service in the memory 250.

In various embodiments, the memory 250 may store a digital assistant client module 257 (hereinafter, referred to as a DA client module), and accordingly store instructions and various user data 258 for performing functions on the client side of the digital assistant.

Meanwhile, the DA client module 257 may obtain voice input, text input, touch input, and/or gesture input of the user via various user interfaces (e.g., I/O subsystem 240) provided in the cardiac output calculating device 200.

In addition, the DA client module 257 may output audio-visual and tactile types of data. For example, the DA client module 257 may output data composed of a combination of at least two of voice, sound, notification, text message, menu, graphic, video, animation, and vibration. In addition, the DA client module 257 may communicate with a digital assistant server (not shown) using the communication subsystem 280.

In various embodiments, the DA client module 257 can collect additional information about the surroundings of the cardiac output calculating device 200 from various sensors, subsystems, and peripheral devices to construct a context associated with the user input. For example, the DA client module 257 can infer a user's intention by providing context information along with a user input to the digital assistant server. Here, the context information that can be accompanied by a user input can include sensor information, such as lighting, ambient noise, ambient temperature, images of the surrounding environment, video, and the like. For another example, the context information can include a physical state (e.g., device orientation, device location, device temperature, power level, speed, acceleration, motion pattern, cellular signal strength, etc.) of the cardiac output calculating device 200. As another example, the context information can include information related to the software status of the cardiac output calculating device 200 (e.g., processes running on the cardiac output calculating device 200, installed programs, past and current network activity, background services, error logs, resource usage, etc.).

In various embodiments, the memory 250 may include additional or deleted instructions, and furthermore, the cardiac output calculating device 200 may include additional configurations other than the configuration illustrated in FIG. 2, or may exclude some configurations.

The processor 220 can control the overall operation of the cardiac output calculating device 200 and can execute various commands for calculating and/or outputting cardiac output of the user by running an application or a program stored in the memory 250.

The processor 220 may correspond to a computing device such as a central processing unit (CPU) or an application processor (AP). In addition, the processor 220 may be implemented in the form of an integrated chip (IC) such as a System on Chip (SoC) in which various computing devices such as a Neural Processing Unit (NPU), are integrated.

In various embodiments, the processor 220 may receive at least one of first bio-signal data including an electrocardiogram and a photoplethysmogram, and demographic information data.

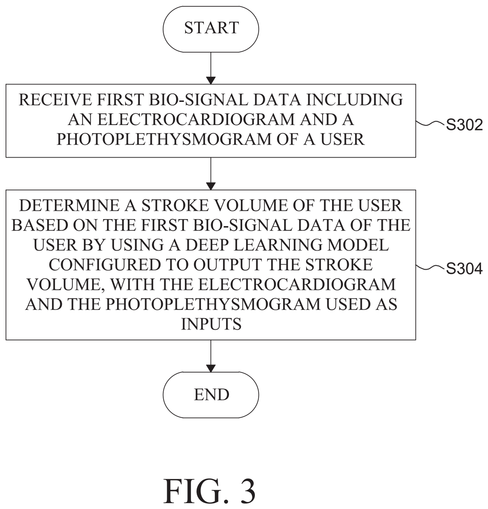

The first bio-signal data may include electrocardiogram waveform data, photoplethysmogram waveform data, and stroke volume data calculated from arterial blood pressure measured using an Arterial Pressure Based Cardiac Output (APCO) equipment. The demographic information data may include at least one of age, sex, height, and weight of the user. The processor 220 may preprocess the first bio-signal data. Further, the processor 220 may extract second bio-signal data from the preprocessed bio-signal data. Further, the processor 220 may infer the stroke volume using a deep learning model configured to output stroke volume with the first bio-signal data and the second bio-signal data as inputs. Details of the operation of the processor 220 will be described below with reference to FIG. 3.

The peripheral interface 230 may be connected to various sensors, subsystems, and peripheral devices to provide data so that the cardiac output calculating device 200 may perform various functions. Here, it may be understood that a function performed by the cardiac output calculating device 200 is performed by the processor 220.

The peripheral interface 230 can receive data from the motion sensor 260, the lighting sensor (light sensor) 261, and the proximity sensor 262 so that the cardiac output calculating device 200 can perform orientation, light, and proximity sensing functions. For another example, the peripheral interface 230 can receive data from other sensors 263 (positioning system-GPS receiver, temperature sensor, biometric sensor), which can allow the cardiac output calculating device 200 to perform functions related to other sensors 263.

In various embodiments, the cardiac output calculating device 200 can include a camera subsystem 270 connected with the peripheral interface 230 and an optical sensor 271 connected thereto, such that the cardiac output calculating device 200 can perform various photographing functions such as photographing and video clip recording.

In various embodiments, the cardiac output calculating device 200 can include communication subsystem 280 connected to the peripheral interface 230. The communication subsystem 280 consists of one or more wired/wireless networks and can include various communication ports, radio frequency transceivers, and optical transceivers.

In various embodiments, the cardiac output calculating device 200 includes an audio subsystem 290 associated with the peripheral interface 230, and such audio subsystem 290 includes one or more speakers 291 and one or more microphones 292, such that the cardiac output calculating device 200 can perform voice-operated functions, such as voice recognition, voice reproduction, digital recording, and telephone functions.

In various embodiments, the cardiac output calculating device 200 can include an I/O subsystem 240 connected to the peripheral interface 230. For example, the I/O subsystem 240 can control the touch screen 243 included in the cardiac output calculating device 200 through the touch screen controller 241. As an example, the touch screen controller 241 can detect a user's contact and movement or cessation of contact and movement by using any one of a plurality of touch sensing technologies such as capacitive, resistive, infrared, surface acoustic wave technology, proximity sensor array, etc. For another example, the I/O subsystem 240 can control other input/control devices 244 included in the cardiac output calculating device 200 through other input controller(s) 242. As an example, the other input controller(s) 242 can control pointer devices such as one or more buttons, a rocker switch, a thumb-wheel, an infrared port, a USB port, and a stylus.

FIG. 3 is a flowchart schematically illustrating a method of calculating cardiac output according to an embodiment of the present disclosure.

Referring to FIG. 3, the cardiac output calculating device 100 (or the transceiver 10 (FIG. 1) according to an embodiment of the present disclosure may receive first bio-signal data including an electrocardiogram and a photoplethysmogram of a user (S302).

The first bio-signal data may include an electrocardiogram waveform, a photoplethysmogram waveform, and a stroke volume calculated from an arterial blood pressure calculated using an Arterial Pressure Based Cardiac Output (APCO) equipment. As an example, an electrocardiogram waveform and a photoplethysmogram waveform measured at a sample speed of 100 Hz, and a stroke volume measured at 2-second intervals may be collected from the publicly available dataset VitalDB.

The cardiac output calculating device 100 (FIG. 1) (or the processor 14 (FIG. 1)) according to an embodiment of the present disclosure may determine a stroke volume of the user based on the first bio-signal data of the user by using a deep learning model configured to output the stroke volume, with the electrocardiogram and the photoplethysmogram used as inputs (S304).

The deep learning model may be a deep learning model learned from training data for a plurality of patients.

The training data for the plurality of patients may include a VPG (first derivative PPG) waveform which is a first-order derivative waveform of the photoplethysmogram, and a APG (second derivative PPG) waveform which is a second-order derivative waveform of the photoplethysmogram. The training data for the plurality of patients may include PR interval, QRS duration, QRS amplitude, and ST segment extracted from the electrocardiogram of each of the plurality of patients. Further, the training data for the plurality of patients may include a Pulse Transit Time (PTT) extracted from the electrocardiogram and the photoplethysmogram of each of the plurality of patients. The training data for a plurality of patients may be referred to as second bio-signal data.

The deep learning model may determine a stroke volume of the user by using the user's first bio-signal data, second bio-signal data, and the demographic information data as inputs. The second bio-signal data may include the VPG waveform, the APG waveform, the PR interval, the QRS duration, the QRS amplitude, the ST segment, and the PTT.

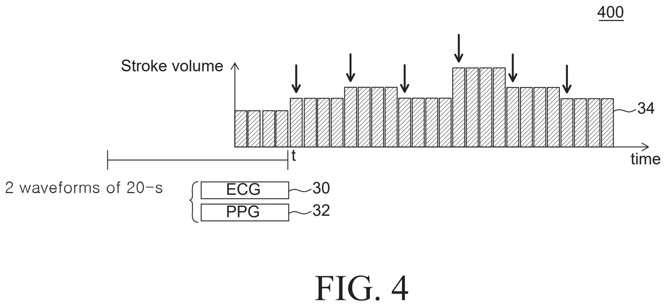

FIG. 4 is a graph illustrating input data and output data of a cardiac output calculating device according to an embodiment of the present disclosure.

Referring to FIG. 4, a graph 400 illustrating input data and output data of a cardiac output calculating device is illustrated. The horizontal axis of the graph 400 is time t, and the vertical axis of the graph 400 is stroke volume.

The input data may be the electrocardiogram (ECG) data 30 and/or photoplethysmogram (PPG) data 32 of the patient. the electrocardiogram (ECG) data (30) and/or photoplethysmogram (PPG) data 32 of the patient may be part of a bio-signal collected during a patient's surgery. The electrocardiogram (ECG) data 30 and/or the photoplethysmogram (PPG) data 32 of the patient may be waveform data for a predetermined period.

For example, the electrocardiogram (ECG) data 30 and/or the photoplethysmogram (PPG) data 32 of the patient may be waveform data spanning 20 seconds. For example, the electrocardiogram (ECG) data 30 and/or photoplethysmogram (PPG) data 32 of the patient for a predetermined period immediately before an arbitrary time t may be used to calculate and output a cardiac output at an arbitrary time t. The predetermined period may be 20 seconds.

For example, the electrocardiogram (ECG) data 30 and/or the photoplethysmogram (PPG) data 32 of the patient may be waveform data spanning 60 seconds. For example, the electrocardiogram (ECG) data 30 and/or photoplethysmogram (PPG) data 32 of the patient for a predetermined period immediately before an arbitrary time t may be used to calculate and output a cardiac output at an arbitrary time t. The predetermined period may be 60 seconds.

The output data may be stroke volume data 34. For example, the output data may be a value at a time point at which the stroke volume changes, such as a time point indicated by an arrow in FIG. 4.

The cardiac output may be calculated by multiplying the stroke volume and the heart rate. The output data of the cardiac output calculating device according to an embodiment of the present disclosure may be a cardiac output. The output data of the cardiac output calculating device according to another embodiment of the present disclosure may be a stroke volume.

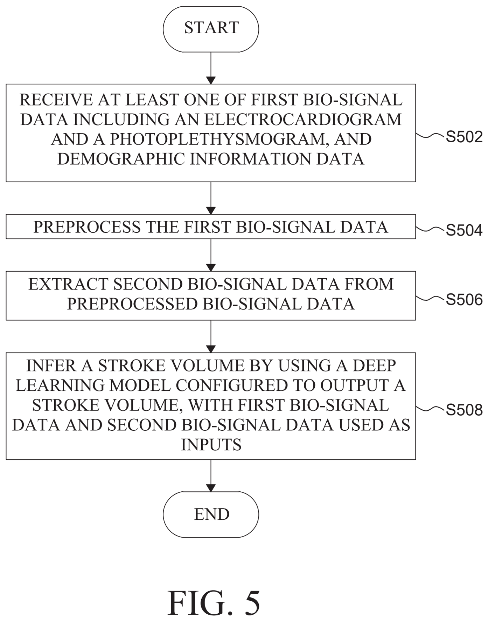

FIG. 5 is a flowchart illustrating a method of calculating cardiac output according to an embodiment of the present disclosure.

Referring to FIG. 5, a cardiac output calculating device 100 (or a transceiver 10 (FIG. 1) according to an embodiment of the present disclosure may receive at least one of first bio-signal data including an electrocardiogram and a photoplethysmogram and demographic information data (S502). The first bio-signal data may include electrocardiogram waveform data, photoplethysmogram waveform data, and stroke volume data calculated from arterial blood pressure calculated using an Arterial Pressure Based Cardiac Output (APCO) equipment. The demographic information data may include at least one of age, sex, height, and weight of the user.

The cardiac output calculating device 100 (FIG. 1) (or the processor 14 (FIG. 1) according to an embodiment of the present disclosure may preprocess the first bio-signal data (S504).

A cardiac output calculating device 100 (FIG. 1) (or a processor 14 (FIG. 1)) may slice the first bio-signal data to a predetermined length, extract sampling data from the sliced first bio-signal data, and remove noise data of the first bio-signal data.

The cardiac output calculating device 100 (FIG. 1) (or the processor 14 (FIG. 1)) may select a sample data set to be used within a predetermined criterion for the collected electrocardiogram waveform data.

As an example, the cardiac output calculating device 100 (FIG. 1) (or the processor 14 (FIG. 1)) may extract a candidate data set of the first reference sample from the collected electrocardiogram waveform data, and the candidate data set of the first reference sample may be used as training data. For example, the first criterion may be 2 or more and 4.5 or less.

As another example, abnormal electrocardiogram waveforms can only be selected if the standard deviation of the RR interval is 30 or less to avoid selection as the dataset to be used. RR interval refers to the interval between R-peaks of the heartbeat.

The cardiac output calculating device 100 (FIG. 1) (or the processor 14 (FIG. 1)) may select a sample data set to be used within a predetermined criterion from the collected photoplethysmogram data.

As an example, the cardiac output calculating device 100 (FIG. 1) (or the processor 14 (FIG. 1)) may extract a candidate data set of the second reference sample from the collected photoplethysmogram waveform data, and the candidate data set of the second reference sample may be used as training data. For example, the second criterion may be 0 or more and 100 or less.

As another example, when the frequency of the photoplethysmogram waveform is less than or equal to a predetermined reference, or when it is determined that the photoplethysmogram waveform is irregular, the photoplethysmogram waveform may be excluded from the sample data set.

As another example, samples with an internal correlation of 0.9 or higher within the bits of the photoplethysmogram may be selected as a candidate data set, and samples with a standard deviation of 0 may be excluded.

The cardiac output calculating device 100 (FIG. 1) (or the processor 14 (FIG. 1)) may perform min-max normalization of the photoplethysmogram.

As to the waveform of the photoplethysmogram measured from the photoplethysmogram measuring device 106, the photoplethysmogram measuring device 106 may autonomously scale the amplitude. Since the scaling factor(s) used when scaling is not known to the user, min-max normalization can be performed to remove inaccurate information from the amplitude of photoplethysmogram.

Further, the cardiac output calculating device 100 (FIG. 1) (or the processor 14 (FIG. 1)) may remove the noise from the signal appearing in the electrocardiogram waveform and/or the photoplethysmogram waveform.

For example, the cardiac output calculating device 100 (FIG. 1) (or the processor 14 (FIG. 1)) may remove a frequency component of 0.5 Hz or less from an electrocardiogram waveform and/or a photoplethysmogram waveform. As another example, frequency components having a cutoff frequency of 0.5 Hz or less and 8 Hz or more may be blocked in the photoplethysmogram waveform.

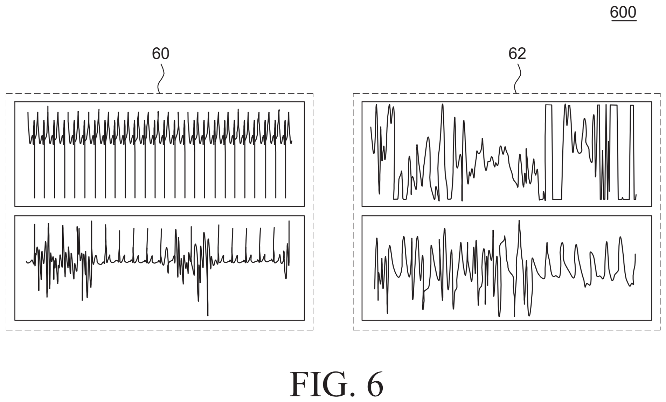

In relation to this, FIG. 6 is a graph illustrating an example of noise of an electrocardiogram waveform and a photoplethysmogram waveform.

Referring to FIG. 6, a graph 600 illustrating an electrocardiogram waveform and a photoplethysmogram waveform received by the cardiac output calculating device 100 (FIG. 1) is illustrated.

A cardiac output calculating device 100 (FIG. 1) (or a transceiver 10 (FIG. 1)) may receive first bio-signal data including electrocardiogram and photoplethysmogram of a plurality of patients measured intraoperatively in an operating room. Due to motion artifact, electrocautery, wall power, etc., the waveform 60 of the electrocardiogram collected in the operating room may include noise data. Due to motion artifact, nerve stimulation, respiration, etc., the waveform 62 of the photoplethysmogram collected in the operating room may include noise data such as baseline drift and the like.

Referring back to S504 of FIG. 5, the preprocessing of the first bio-signal data may include removing noise from a signal appearing in an electrocardiogram waveform and/or a photoplethysmogram waveform that interferes with the training of the cardiac output calculation model 16 (see FIG. 1).

The cardiac output calculating device 100 (FIG. 1) (or the processor 14 (FIG. 1)) may select a sample data set to be used within a predetermined criterion for the collected stroke volume data.

For example, the cardiac output calculating device 100 (FIG. 1) (or the processor 14 (FIG. 1)) may extract a candidate data set of the third reference sample from the collected stroke volume data, and the candidate data set of the third reference sample may be used as training data. For example, the third criterion may be 20 mmHg or more and 200 mmHg or less.

Since the stroke volume calculated using the APCO equipment is information calculated based on arterial blood pressure, the recorded value of arterial blood pressure may be recorded without being placed in the artery or may be a value due to an artifact due to a flush of the arterial internal pipe, so selecting according to the third criterion is to prevent this.

A cardiac output calculating device 100 (FIG. 1) (or the processor 14 (FIG. 1)) according to an embodiment of the present disclosure may extract second bio-signal data from preprocessed bio-signal data (S506).

The cardiac output calculating device 100 (FIG. 1) (or the processor (14, FIG. 1)) according to an embodiment of the present disclosure may extract a VPG (first derivative PPG) waveform which is a first-order derivative waveform of the photoplethysmogram, and a APG (second derivative PPG) waveform which is a second-order derivative waveform of the photoplethysmogram from the preprocessed photoplethysmogram and use it as a secondary index when calculating cardiac output. The VPG waveform and/or the APG waveform may be referred to as 'second bio-signal data'.

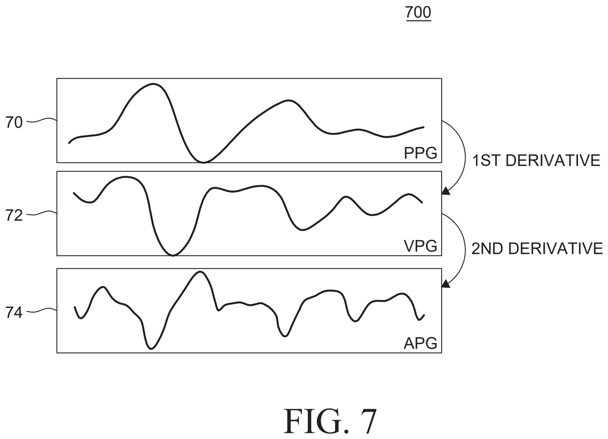

In relation to this, FIG. 7 is a graph illustrating a secondary index extracted from a photoplethysmogram waveform and a photoplethysmogram waveform.

Referring to FIG. 7, graphs illustrating the photoplethysmogram (PPG) waveform 70, the VPG waveform 72, and the APG waveform 74 are illustrated.

In order to compensate for insufficient information by eliminating inaccurate information on the amplitude of the waveform of the photoplethysmogram by performing min-max normalization of the photoplethysmogram waveform, the cardiac output calculating device 100 (FIG. 1) (or a processor (14, FIG. 1)) according to an embodiment of the present disclosure may further utilize the VPG waveform 72 and/or the APG waveform 74 as well as the photoplethysmogram (PPG) waveform 70 when calculating cardiac output.

Referring back to S506 of FIG. 5, the cardiac output calculating device 100 (or the processor 14 (FIG. 1) according to an embodiment of the present disclosure may extract PR interval, QRS duration, QRS amplitude, and ST segment from the preprocessed electrocardiogram waveform and use the same as a secondary index when calculating cardiac output.

In relation to this, FIG. 7 is a graph illustrating an electrocardiogram waveform and a secondary index extracted from the electrocardiogram waveform.

Referring to FIG. 7, graphs illustrating electrocardiogram waveforms 800, PR interval, QRS duration, QRS amplitude, and ST segment are shown.

PR interval can be defined as the time interval from the beginning of the P wave to the beginning of the QRS complex on the electrocardiogram, and it means the time when neurotransmitter occurs in the heart. QRS duration can be defined as the time it takes for the QRS complex to appear on the electrocardiogram, and it means the time the heart contracts. The QRS amplitude represents the maximum bipolar difference in the QRS complex, and the state of the heart can be diagnosed by the intensity of electrical activity occurring in the heart. The ST segment can be defined as the time interval from the end of the QRS complex to the start point of the T wave, and the condition of the heart can be diagnosed by the bipolar changes that occur between the contraction and relaxation of the heart.

The cardiac output calculating device 100 (FIG. 1) (or the processor 14 (FIG. 1)) according to an embodiment of the present disclosure may additionally utilize a PR interval, a QRS duration, a QRS template, and an ST segment as well as an electrocardiogram waveform 800 when calculating cardiac output. For example, a cardiac output calculation model 16 (FIG. 1) may train characteristics of heart contraction using secondary indices extracted from electrocardiogram (ECG).

Referring back to S506 of FIG. 5, the cardiac output calculating device 100 (or the processor 14 (FIG. 1)) according to an embodiment of the present disclosure may extract Pulse Transit Time (PTT) from the preprocessed electrocardiogram waveform and photoplethysmogram and use the same as a secondary index when calculating cardiac output.

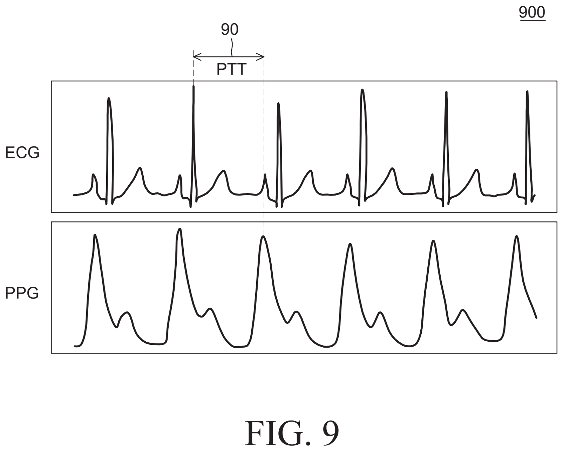

In relation to this, FIG. 9 is a graph illustrating secondary indices extracted from electrocardiogram waveforms and photoplethysmogram waveforms.

Referring to FIG. 9, a graph illustrating an electrocardiogram (ECG) waveform, a photoplethysmogram (PPG) waveform, and a PTT 90 is illustrated.

Pulse Transit Time (PTT) can be defined as the time between the peak point of the QRS complex and a specific reference point on the PPG waveform measured at the finger tip . PTT can be interpreted as the occurrence time of a pulse, i.e., the time the blood contracts from the heart and is delivered to the vascular measurement part of the peripheral part. PTT is an index used to more efficiently analyze and evaluate blood pressure, and can be used as an index indicating the elasticity of blood vessels.

The cardiac output calculating device 100 (FIG. 1) (or the processor 14 (FIG. 1)) according to an embodiment of the present disclosure may further utilize the PTT 90 as well as the electrocardiogram (ECG) waveform and the photoplethysmogram waveform (PPG) when calculating cardiac output.

Referring back to FIG. 5, the cardiac output calculating device 100 (or the processor 14 (FIG. 1)) according to an embodiment of the present disclosure may infer a stroke volume by using a deep learning model configured to output a stroke volume, with first bio-signal data and second bio-signal data used as inputs (S508).

The processor 14 (FIG. 1) of the cardiac output calculating device 100 (FIG. 1) may match and store demographic information including the electrocardiogram waveform, photoplethysmogram waveform, secondary indices extracted from electrocardiogram waveform, and/or photoplethysmogram waveform, the stroke volume information, age, sex, and height weight for a particular patient. The above-described saved data may be used as training data.

The processor 14 (FIG. 1) of the cardiac output calculating device 100 (FIG. 1) may train the cardiac output calculation model 16 (FIG. 1). The cardiac output calculation model 16 (FIG. 1) may be a deep learning model having a weight value obtained by extracting and combining various features.

The processor 14 (FIG. 1) of the cardiac output calculating device 100 (FIG. 1) may smooth training data. The smoothed training data may be entered into a cardiac output calculation model 16 (FIG. 1), and the cardiac output calculation model 16 (FIG. 1) may be trained by extracting and combining various features to update the weights.

A cardiac output calculating device 100 (FIG. 1) (or the processor 14, (FIG. 1)) can infer a stroke volume of a specific patient using biometric data including electrocardiogram and photoplethysmogram, stroke volume information, demographic information including age, sex, and height weight, and a Cardiac Output Calculation Model 16 (FIG. 1).

FIG. 10 is a block diagram illustrating an architecture of a Cardiac Output Calculation Model according to an embodiment of the present disclosure.

Referring to FIGS. 1 and 10, the cardiac output calculation model 1000 may be applied to the cardiac output calculation model 16 of FIG. 1.

Referring to FIG. 10, the input data of the cardiac output calculation model 1000 may include bio-signal data 1002 including a waveform of an electrocardiogram, a waveform of a photoplethysmogram, a VPG waveform, and an APG waveform, and demographic data 1004 including age, sex, and height.

Bio signal data 1002 input to the cardiac output calculation model 1000 may be continuous data that is sliced for a predetermined period. The predetermined period may be referred to as a 'sliding window'. The patient's electrocardiogram waveform and/or photoplethysmogram waveform can be input into the cardiac output calculation model 1000 by sliding over a predetermined sliding window (e.g., 20 seconds).

The input data of the cardiac output calculation model 1000 may include extracted features 1006 extracted from a waveform of an electrocardiogram and/or a waveform of a photoplethysmogram.

The data 1006 extracted from the waveform of the electrocardiogram and/or the waveform of the photoplethysmogram may include PR interval, QRS duration, QRS amplitude, ST segment extracted from the electrocardiogram waveform, and PTT extracted from the electrocardiogram waveform and the photoplethysmogram. The data 1006 extracted from the waveform of the electrocardiogram and/or the waveform of the photoplethysmogram input to the cardiac output calculation model 1000 may be an average of values calculated for a predetermined period. For example, an average value of each of the PR interval, QRS duration, QRS amplitude, ST segment, and PTT values calculated for 20 seconds or 60 seconds may be input to the cardiac output calculation model 1000.

Bio-signal data 1002 including an electrocardiogram waveform, a photoplethysmogram waveform, a VPG waveform, and an APG waveform may be normalized for training the cardiac output calculation model 1000. In addition, Demographics 1004, data 1006 extracted from the electrocardiogram waveform and/or the photoplethysmogram waveform may be normalized for training the cardiac output calculation model 1000. Accordingly, in the cardiac output calculation model 1000, bio-signal data 1002, demographics 1004, and data 1006 extracted from a waveform of an electrocardiogram and/or a waveform of a photoplethysmogram may be normalized and input.

The cardiac output calculation model 1000 according to an embodiment of the present disclosure may be trained to more accurately predict cardiac output by using the high-resolution bio-signal data 1002. In addition, by extracting and utilizing secondary indices related to cardiac output (e.g., APG waveform, VPG waveform, PR interval, QRS duration, QRS amplitude, ST segment, PTT) from high-resolution electrocardiogram and photoplethysmogram waveforms, the performance of the cardiac output calculation model 1000 can be improved.

The cardiac output calculation model 1000 according to an embodiment of the present disclosure may have a Convolution Neural Network (CNN) structure including at least one Squeeze and Execution (SE) block and at least one Residual block.

For example, the cardiac output calculation model 1000 may include a convolutional neural network having an SE-ResNet structure.

ResNet may be defined as an algorithm that solves the vanishing gradient problem that may occur as the layer deepens by training the residual, which is the difference between the input and output of each layer. The SE may be defined as a module that performs an operation of squeezing and exciting information. The SE is for recalibration by calculating the importance of each channel and weighting important channels in consideration of the inter-channel dependence of the characteristic map, which is the result of passing through each layer.

The cardiac output calculation model 1000 may perform convolution using a convolution filter of various sizes while passing through a plurality of layers, and the residual learning may simultaneously be performed. In addition, in the cardiac output calculation model 1000, Max pooling and/or global average pooling (GAP) may be used as a compression (Squeeze) operation.

The cardiac output calculation model 1000 may infer a stroke volume of a specific patient by using the above-described input data 1002, 1004, and 1006 and the learned CNN structure including the SE block and the residual block. The output data 1008 of the cardiac output calculation model 1000 may be a stroke volume. For example, the cardiac output calculation model 1000 may output a single output every 8 seconds.

The cardiac output calculation model 1000 may correspond to an algorithm of any structure including a convolutional neural network, and the technical scope of the present disclosure is not limited or limited by the above-described embodiment of the cardiac output calculation model 1000.

According to one embodiment of the present disclosure, a data set used for the development and learning of a cardiac output calculation model 1000 includes biometric data including electrocardiogram and photoplethysmogram collected during 3,970 operations at a particular hospital, stroke volume information, demographic information including age, sex, and height and weight.

Specifically, data sets collected by the Time series hold-out method during 3,970 surgeries at specific hospitals were divided into training data sets, validation data sets, and test data sets. The training data set, verification data set, and test data set consisted of a ratio of 6:2:2. The training dataset includes 741,150 samples collected during 2,777 surgeries, and the test dataset includes 196,686 samples collected during 694 surgeries.

In order to evaluate the validity of the stroke volume (i.e., a predicted value) output from the Cardiac Output Calculation Model and the stroke volume (i.e., an actual value) calculated based on the arterial blood pressure, the Cardiac Output Calculation Model according to an embodiment of the present disclosure uses a Percentage Error, a Mean Absolute Percentage Error (MAPE), and a Mean Absolute Error (MAE).

The percentage error can be interpreted as an index to assess whether the change in the difference between the actual value and the predicted value is acceptable.

The mean absolute percentage error (MAPE) is the average of the errors between the actual and predicted values, calculated as a percentage, and is a value expressed as a ratio of MAE. For example, if the MAE value of the Cardiac Output Calculation Model is 12 ml/beat, it means that the model incorrectly predicted 12 ml/beat on average.

Mean absolute error (MAE) means the average of the absolute error between the actual value and the predicted value. For example, when the MAPE of the Cardiac Output Calculation Model is 12%, it means that the ratio of the actual value to the predicted value differs by about 12%.

Percentage Error, MAPE, and MAE show better performance at lower values.

The performance evaluation result of the Cardiac Output Calculation Model according to an embodiment of the present disclosure may be expressed as shown in Table 1 below.

[Table 1]

| | Percentage error of 10min median (%) (10min interval) | Percentage Error (%) | MAPE (%) | MAE (ml/beat) |
|---|---|---|---|---|
| RESULT | 37.6 | 41.5 | 16.1 | 12.2 |

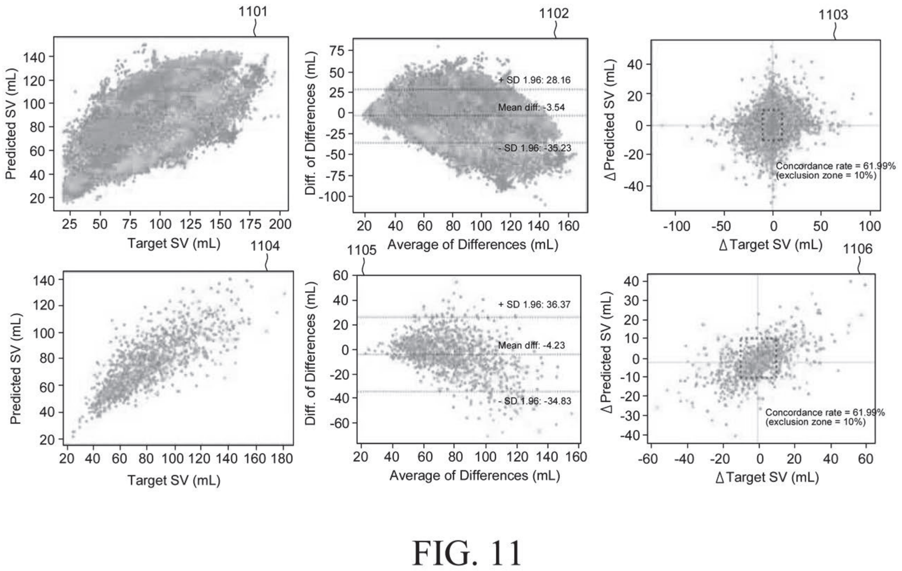

Referring to Table 1, since stroke volume is not a value that actually changes frequently, a median filter was applied to the predicted values and actual values measured at 10-minute intervals to calculate the percentage error, and the percentage error was measured to be 37.6%. FIG. 11 shows graphs comparing the predicted values and actual values of the Cardiac Output Calculation Model according to an embodiment of the present invention.

Referring to FIG. 11, graphs 1101, 1102, and 1103 for continuous cardiac output and graphs 1104, 1105, and 1106 for cardiac output measured at intervals of 10 minutes are illustrated.

Referring to FIG. 11, various indices of an actual value and a predicted value are compared in the form of scatter plots 1101 and 1104, Bland-Altman plots 1102 and 1105, and four quadrate plots 1103 and 1106.

Referring to the scatter plot (1101, 1104), it can be seen that there is a linear relationship between the continuous cardiac output (Target SV(mL)) calculated based on arterial blood pressure and the cardiac output (Predicted SV(mL)) output from the Cardiac Output Calculation Model.

Referring to the Bland-Altman plots 1102 and 1105, it may be seen that most of the points are located around the bias.

Bland-Altman plot can be defined as a scatter plot that calculates the mean of and difference between each pair of measurements from two sets of measurements of the same subject (stroke volume calculated based on arterial blood pressure and stroke volume output from the Cardiac Output Calculation Model). The x-axis of the Bland-Altman plot represents the average (Average of Differences(mL)), and the y-axis represents the difference (Diff.of Differences(mL)).

Three horizontal dotted lines parallel to the x-axis may be displayed on the Bland-Altman plot, which may be interpreted as values summarizing the degree of discrepancy. Among the three horizontal dotted lines, the upper line represents the 95% upper limit of agreement, the center line represents the difference from the mean, and the lower line represents the 95% lower limit of agreement. According to an embodiment of the present disclosure, the percentage error may be calculated as in Equation 1 below.

[Equation 1]

$$\text{Percentage Error} = 100*((1.96*SD)/\text{mean of Stroke volume})$$

Referring to the four quadrate plot 1106 for the cardiac output measured at intervals of 10 minutes, it may be seen that the consistency rate between the actual value and the predicted value is 79.2%, and the ratio of points having a positive correlation is high.

Although the embodiments of the present invention have been described in more detail with reference to the attached drawings, the present invention is not necessarily limited to these embodiments, and various modifications can be made without departing from the technical idea of the present invention. Accordingly, the embodiments disclosed in the present invention are not intended to limit the technical idea of the present invention, but to explain it, and the scope of the technical idea of the present invention is not limited by these embodiments. Therefore, it should be understood that the embodiments described above are exemplary in all aspects and not restrictive. The protection scope of the present invention should be interpreted by the claims below, and all technical ideas within the equivalent scope should be interpreted as being included in the scope of the rights of the present invention.

**Claims**

1.    A method of operating a device for non-invasively calculating cardiac output, comprising:

receiving first bio-signal data including an electrocardiogram and a photoplethysmogram of a user; and
determining a stroke volume of the user on the basis of the first bio-signal data of the user by using a deep learning model configured to output the stroke volume, with the electrocardiogram and the photoplethysmogram used as inputs.

2.    The method of claim 1,

the deep learning model is trained from training data for a plurality of patients, and
the training data for the plurality of patients includes a VPG (first derivative PPG) waveform which is a first-order derivative waveform of the photoplethysmogram, and a APG (second derivative PPG) waveform which is a second-order derivative waveform of the photoplethysmogram.

3. The method of claim 2,
the training data for the plurality of patients includes PR interval, QRS duration, QRS amplitude, and ST segment extracted from the electrocardiogram of each of the plurality of patients.

4. The method of claim 3,
the training data for the plurality of patients includes a Pulse Transit Time (PTT) extracted from the electrocardiogram and the photoplethysmogram of each of the plurality of patients.

5. The method of claim 4, further comprising:

receiving the user's demographic information data,
the deep learning model is configured to determine a stroke volume of the user by using the user's first bio-signal data, second bio-signal data, and the demographic information data as inputs,
the demographic information data include at least one of the user's age, sex, height, and weight, and
the second bio-signal data include the VPG waveform, the APG waveform, the PR interval, the QRS duration, the QRS amplitude, the ST segment, and the PTT.

6. A device for non-invasively calculating cardiac output, comprising:

a memory;
a transceiver receiving at least one of a first bio-signal data including an electrocardiogram and a photoplethysmogram, and the demographic information data; and
a processor including a deep learning model;
the processor is configured to preprocess the first bio-signal data, extract second bio-signal data from the preprocessed bio-signal data, and infer a stroke volume using a deep learning model configured to output stroke volume by using the first bio-signal data and the second bio-signal data as inputs.

7. The device of claim 6,
the processor is further configured to slice the first bio-signal data into a predetermined length, extract sampling data from the sliced first bio-signal data, and remove noise data from the first bio-signal data.

8. The device of claim 7,
the second bio-signal data includes at least one of a VPG (first derivative PPG) waveform which is a first-order derivative waveform of the photoplethysmogram, a APG (second derivative PPG) waveform which is a second-order derivative waveform of the photoplethysmogram, and a PR interval, QRS duration, QRS amplitude, ST segment extracted from the electrocardiogram, and a Pulse Transit Time (PTT) extracted from the electrocardiogram and the photoplethysmogram.

9. The device of claim 8,
the processor is further configured to perform a min-max normalization of the photoplethysmogram and extract the VPG waveform and the APG waveform of the photoplethysmogram, and the VPG waveform and the APG waveform is input to the deep learning model in the form of time series data having the predetermined length.

10. The device of claim 8,
the PR interval, the QRS duration, the QRS amplitude, the ST segment, and the PTT is input to the deep learning model as an average of each value measured multiple times during a time corresponding to the predetermined length.

11. The device of claim 6,
the training data includes the first bio-signal data, the second bio-signal data, stroke volume data, and the demographic information data of a plurality of patients, and the demographic information data includes at least one of age, sex, height, and weight.

12. The device of claim 11,

the memory is configured to store a set of training data matched for each of the plurality of patients.

13. The device of claim 6,
the deep learning model has a CNN (configuration Neural Network) structure including at least one SE (Squeeze and Excitation) block and at least one Residual block.

14. The device of claim 6, comprising:

the transceiver which are further configured to receive the first bio-signal data and the second bio-signal data of the user corresponding to a period from t(t is a positive integer) seconds to (t+n) seconds (n is a positive integer), and
the processor which are further configured to calculate the stroke volume of the user at the (t+n) seconds by using the deep learning model.

FIG. 1

200

CARDIAC OUTPUT CALCULATING DEVICE

250 — MEMORY
251 — OPERATING SYSTEM
252 — COMMUNICATION MODULE
253 — GUI MODULE
254 — SENSOR PROCESSING MODULE
255 — TELEPHONE MODULE
256 — APPLICATION MODULE
256-1 — APPLICATION
256-2 — APPLICATION
257 — DIGITAL ASSISTANT CLIENT MODULE
258 — USER DATA

OTHER SENSOR(S) — 263
MOTION SENSOR — 260
LIGHTING SENSOR — 261
PROXIMITY SENSOR — 262

210 — MEMORY INTERFACE

PERIPHERAL INTERFACE

CAMERA SUBSYSTEM — 270 | OPTICAL SENSOR — 271
COMMUNICATION SUBSYSTEM(S) — 280
AUDIO SUBSYSTEM — 290 — 291
— 292

220 — PROCESSOR(S)

230

240 — I/O SUBSYSTEM

TOUCH SCREEN CONTROLLER

OTHER INPUT CONTROLLER(S)

241 — TOUCH SCREEN — 243

OTHER INPUT/CONTROL DEVICES — 242

244

FIG. 2

START

RECEIVE FIRST BIO-SIGNAL DATA INCLUDING AN ELECTROCARDIOGRAM AND A PHOTOPLETHYSMOGRAM OF A USER —— S302

DETERMINE A STROKE VOLUME OF THE USER BASED ON THE FIRST BIO-SIGNAL DATA OF THE USER BY USING A DEEP LEARNING MODEL CONFIGURED TO OUTPUT THE STROKE VOLUME, WITH THE ELECTROCARDIOGRAM AND THE PHOTOPLETHYSMOGRAM USED AS INPUTS —— S304

END

# FIG. 3

FIG. 4

START

RECEIVE AT LEAST ONE OF FIRST BIO-SIGNAL
DATA INCLUDING AN ELECTROCARDIOGRAM
AND A PHOTOPLETHYSMOGRAM, AND
DEMOGRAPHIC INFORMATION DATA ⟩S502

PREPROCESS THE FIRST BIO-SIGNAL DATA ⟩S504

EXTRACT SECOND BIO-SIGNAL DATA FROM
PREPROCESSED BIO-SIGNAL DATA ⟩S506

INFER A STROKE VOLUME BY USING A DEEP
LEARNING MODEL CONFIGURED TO OUTPUT A
STROKE VOLUME, WITH FIRST BIO-SIGNAL
DATA AND SECOND BIO-SIGNAL DATA USED AS
INPUTS ⟩S508

END

# FIG. 5

FIG. 6

700

FIG. 7

800

QRS duration

R

QRS amplitude

T

P

Q  S

ST segment

PR interval

FIG. 8

FIG. 9

EP 4 728 980 A1

PPG

ECG

APG

VPG

Normalization

$$X_{normalized} = \frac{(X - Xmin)}{(Xmax - Xmin)}$$

1000

Bio signal

Input (N, 2000, 4) — 1002

ConV1D (64), k=7
Max pooling, k=3, s=2

ConV1D (64), k=3
ConV1D (64), k=3

⊕

ConV1D (64), k=3
ConV1D (64), k=3

⊕

ConV1D (128), k=3, s=2
ConV1D (128), k=3
Attention module

⊕

ConV1D (128), k=3
ConV1D (128), k=3
Attention module

⊕

GAP

1004
Demographics
Input (N, 4)

Fully connected (4)

1006
Extracted Features
Input (N, 5)

Fully connected (16)

Fully connected (128)

Input (N, 1) — 1008

FIG. 10

FIG. 11

## INTERNATIONAL SEARCH REPORT

| International application No. |
|---|
| **PCT/KR2023/019829** |

| A. | CLASSIFICATION OF SUBJECT MATTER |
|---|---|

**A61B 5/029**(2006.01)i; **A61B 5/00**(2006.01)i; **G16H 50/70**(2018.01)i; **G16H 50/20**(2018.01)i; **G06N 3/08**(2006.01)i; **A61B 5/021**(2006.01)i; **A61B 5/349**(2021.01)i; **A61B 5/358**(2021.01)i; **A61B 5/36**(2021.01)i

According to International Patent Classification (IPC) or to both national classification and IPC

| B. | FIELDS SEARCHED |
|---|---|

Minimum documentation searched (classification system followed by classification symbols)

A61B 5/029(2006.01); A61B 5/00(2006.01); A61B 5/0205(2006.01); A61B 5/024(2006.01); A61B 5/053(2006.01); A61B 5/08(2006.01); A61B 5/085(2006.01); A61B 5/1455(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 심박출량(cardiac output), 심전도(electrocardiogram), 광용적맥파 (photoplethysmography), 전처리(preprocessing), 딥러닝(deep learning)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2013-0107066 A (NUGA MEDICAL CO., LTD. et al.) 01 October 2013 (2013-10-01)<br>See paragraphs [0034], [0039], [0081], [0091] and [0118]; claims 1-3; and figures 1-2. | 1-14 |
| A | KR 10-2018-0040400 A (SAMSUNG ELECTRONICS CO., LTD.) 20 April 2018 (2018-04-20)<br>See paragraphs [0038]-[0064]; and figures 1a-5b. | 1-14 |
| A | KR 10-2021-0003606 A (UNIVERSITY OF ULSAN FOUNDATION FOR INDUSTRY COOPERATION et al.) 12 January 2021 (2021-01-12)<br>See paragraphs [0031]-[0061]; and figures 1-2. | 1-14 |
| A | WO 2017-032873 A2 (RESMED SENSOR TECHNOLOGIES LIMITED) 02 March 2017 (2017-03-02)<br>See paragraphs [0045]-[0081]. | 1-14 |
| A | US 2017-0049391 A1 (MELKER, Richard J.) 23 February 2017 (2017-02-23)<br>See paragraphs [0012]-[0018]. | 1-14 |

| ✓ Further documents are listed in the continuation of Box C. | ✓ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 March 2024** | **08 March 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/019829** |

| C. | DOCUMENTS CONSIDERED TO BE RELEVANT | |
| --- | --- | --- |
| Category* | Citation of document, with indication, where appropriate, of the relevant passages | Relevant to claim No. |
| A | PARK, Seong-A et al. Development of an AI model for non-invasive prediction of cardiac output. YouTube. [online]. [video]. Seoul National University Hospital. 2022 SG Healthcare AI Datathon. 04 December 2022. [Retrieved on 20 February 2024]. Retrieved from <https://www.youtube.com/watch?v=quzvUkVAxBM>.<br><br>  See entire document.<br><br>  ※ This document is a known document declaring exceptions to lack of novelty by the applicant. | 1-14 |

Form PCT/ISA/210 (second sheet) (July 2022)

| International application No.
PCT/KR2023/019829 |

| Patent document cited in search report | | | Publication date (day/month/year) | Patent family member(s) | | | Publication date (day/month/year) |
|---|---|---|---|---|---|---|---|
| KR | 10-2013-0107066 | A | 01 October 2013 | KR | 10-1402134 | B1 | 11 June 2014 |
| | | | | WO | 2013-141419 | A1 | 26 September 2013 |
| KR | 10-2018-0040400 | A | 20 April 2018 | CN | 107928643 | A | 20 April 2018 |
| | | | | CN | 107928643 | B | 01 April 2022 |
| | | | | EP | 3308701 | A1 | 18 April 2018 |
| | | | | EP | 3308701 | B1 | 03 August 2022 |
| | | | | JP | 2018-061826 | A | 19 April 2018 |
| | | | | JP | 2021-154152 | A | 07 October 2021 |
| | | | | JP | 6904796 | B2 | 21 July 2021 |
| | | | | JP | 7183342 | B2 | 05 December 2022 |
| | | | | US | 10820858 | B2 | 03 November 2020 |
| | | | | US | 11666277 | B2 | 06 June 2023 |
| | | | | US | 2018-0098731 | A1 | 12 April 2018 |
| | | | | US | 2021-0085246 | A1 | 25 March 2021 |
| KR | 10-2021-0003606 | A | 12 January 2021 | KR | 10-2281745 | B1 | 26 July 2021 |
| | | | | WO | 2021-002656 | A1 | 07 January 2021 |
| WO | 2017-032873 | A2 | 02 March 2017 | CN | 108135534 | A | 08 June 2018 |
| | | | | CN | 108135534 | B | 05 April 2022 |
| | | | | CN | 114588445 | A | 07 June 2022 |
| | | | | EP | 3340876 | A2 | 04 July 2018 |
| | | | | EP | 3838138 | A2 | 23 June 2021 |
| | | | | EP | 3838138 | A3 | 15 September 2021 |
| | | | | JP | 2018-531055 | A | 25 October 2018 |
| | | | | JP | 2021-180853 | A | 25 November 2021 |
| | | | | JP | 6987042 | B2 | 22 December 2021 |
| | | | | JP | 7284782 | B2 | 31 May 2023 |
| | | | | US | 2020-0297955 | A1 | 24 September 2020 |
| | | | | WO | 2017-032873 | A3 | 13 April 2017 |
| | | | | WO | 2017-032873 | A4 | 13 July 2017 |
| US | 2017-0049391 | A1 | 23 February 2017 | EP | 1883349 | A2 | 06 February 2008 |
| | | | | EP | 1883349 | A4 | 08 April 2015 |
| | | | | EP | 1883349 | B1 | 16 December 2020 |
| | | | | JP | 2008-538936 | A | 13 November 2008 |
| | | | | JP | 5284082 | B2 | 11 September 2013 |
| | | | | US | 2006-0241506 | A1 | 26 October 2006 |
| | | | | US | 2008-0190430 | A1 | 14 August 2008 |
| | | | | US | 2015-0101609 | A1 | 16 April 2015 |
| | | | | US | 7785262 | B2 | 31 August 2010 |
| | | | | US | 8801620 | B2 | 12 August 2014 |
| | | | | US | 9370634 | B2 | 21 June 2016 |
| | | | | US | 9974479 | B2 | 22 May 2018 |
| | | | | WO | 2006-116469 | A2 | 02 November 2006 |
| | | | | WO | 2006-116469 | A3 | 05 July 2007 |

Form PCT/ISA/210 (patent family annex) (July 2022)